# EUROPEAN PATENT SPECIFICATION

(11) **EP 1 814 904 B1**
(45) Date of publication and mention of the grant of the patent: **12.01.2011**
(21) Application number: 05810790.5
(22) Date of filing: 10.11.2005
(51) Int. Cl.: C07K 14/435, A61K 38/17

(54) **ANTIBIOTIC DROSOCIN DERIVATIVES**
ANTIBIOTISCHE DROSOCINDERIVATE
DÉRIVÉS ANTIBIOTIQUES DE LA DROSOCINE

(30) Priority: 10.11.2004 EP 04078089
(43) Date of publication of application: 08.08.2007
(73) Proprietor: Nederlandse Organisatie voor Toegepast -Natuurwetenschappelijk Onderzoek TNO, 2628 VK Delft (NL)
(72) Inventor: VAN HOOFT, Petrus, Albertus, Venantia, NL-2272 XV Voorburg (NL); NOORT, Daniel, NL-2631 VC Nootdorp (NL); DE VISSER, Peter, Christian, NL-2315 KL Leiden (NL); OVERKLEEFT, Herman, Steven, NL-2312 VL Leiden (NL); VAN BOOM, Jacobus, Hubertus, NL-2343 CR Oegstgeest (NL); BIKKER, Floris, Jacob, NL-2707 HW Gouda (NL)
(74) Representative: van Loon, C.J.J.
(86) International application number: PCT/NL2005/000790
(87) International publication number: WO 2006/052129

(56) References cited:
- WO-A-00/17226
- WO-A-94/05787
- WO-A-99/64611
- US-B2- 6 503 881
- CUDIC M ET AL.: "In vitro and in vivo activity of an antibacterial peptide analog against uropathogens" PEPTIDES, vol. 24, 2003, pages 807-820, XP002329691
- GOBBO M ET AL.: "Antimicrobial Peptides: Synthesis and Antibacterial Activity of Linear and Cyclic Drosocin and Apidaecin 1b Analogues" JOURNAL OF MEDICINAL CHEMISTRY, vol. 45, no. 20, 2002, pages 4494-4504, XP002329692
- BENCIVENGO A-M ET AL.: "The efficacy of the antibacterial peptide, pyrrhocoricin, is finely regulated by its amino acid residues and active domains" LETTERS IN PEPTIDE SCIENCE, vol. 8, 2002, pages 201-209, XP002329693
- DE VISSER P C ET AL: "Biological evaluation of Tyr6 and Ser7 modified drosocin analogues" BIOORGANIC & MEDICINAL CHEMISTRY LETTERS, OXFORD, GB, vol. 15, no. 11, 2 June 2005 (2005-06-02), pages 2902-2905, XP004906920 ISSN: 0960-894X
- OTVOS L ET AL: "Insect peptides with improved protease-resistance protect mice against bacterial infection" PROTEIN SCIENCE, vol. 9, 1 January 2000 (2000-01-01), pages 742-749,
- "keyword: pH" ROEMPP Online, Version 3.5 Retrieved from the Internet: URL:www.roempp.com

## Description

### FIELD OF THE INVENTION

The invention relates to the field of antibiotics, more specifically to improved proline rich antibiotics, especially drosocin derivatives.

### INTRODUCTION

The ever increasing number of multi-drug resistant pathogens has urged the need for new antibiotics. In the last decade scientists have put much effort in the developments of novel antibacterial agents as well as improvement of current chemotherapeutic agents. Interestingly, many mammals and insects are remarkably resistant to bacterial infection, due to their ability to produce small-sized cationic peptides. This form of protection, better known as the innate immune system, is highly selective in killing the invader. Many scientists have focused their attention on these antimicrobial peptides (AMP) as they are currently regarded as an important pool of potentially novel antibiotics.

Thus far, many types of antimicrobial peptides have been isolated and sequences from various sources during past decades (for selected reviews, see: Otvos Jr., L. Cell. Mol. Life Sci. 2002, 59:1138; Otvos, Jr., L. J. Peptide Sci. 2000, 6:497; Tan, Y.-T. et al., Mol. Med. Today 2000, 6:309; Scott, M.G. and Hancock, R.E.W., Crit. Rev. Immunol. 2000, 20:407; Hancock, R.E.W. and Chapple, D.S. Antimicrob. Agents Chemother. 1999, 43:1317; Hetru, C. et al., In: Molecular Mechanisms of Immune Responses in Insects; Brey, P. and Hultmark, D. Ed., Chapman and Hall, London, 1998, pp. 40-66; Hancock, R.E.W. et al., Adu. Microb. Physiol. 1995 37:135; Vaara, M. Microbiol. Rev. 1992, 395). Based on their typical primary and secondary structure these peptides can be classified into four main classes, namely 1) the α-helical peptides (magainins and cecropins) (Steiner, H. et al., Nature 1981, 292:246; Zasloff, M. Proc. Natl. Acad. Sci. USA 1987, 5449), 2) the β-sheet peptides containing two or three intramolecular disulfide bridges (defensins) (Hoffman, J. and Hetru, C, Immunol. Today 1992, 13:411); 3) glycine rich peptides (Dimarcq, J.-L. et al., Eur. J. Biochem. 1988, 171:17) end 4) the proline-rich peptides (Bulet, P. et al., J. Biol. Chem. 1993, 268:14983; Coniancich, S. et al., Biochem. J. 1994, 300:567).

Drosocin is a glycopeptide antibiotic that has been isolated from *Drosophila.* It belongs to a family of insect derived proline rich antibiotics, which also includes formaecin, pyrrhocoricin, apideacin, abaecin, metchnikowin, lebocin and diptericin (J.P. Gillespie et al., 1997, Annu. Rev. Entomol. 42: 611-643). It is a proline rich peptide of only 19 amino acids, of which 6 are proline residues:
Gly-Lys-**Pro**-Arg-**Pro**-Tyr-Ser*-**Pro**-Arg-**Pro**-Thr-Ser-His-**Pro**-Arg-**Pro**-Ile-Arg-Val
   * Glycosylated at the Serine residue with Gal(β1→3)GalNac(α1→O)

The proline-rich peptides do not merely kill bacteria by permeabilizing their membranes, but bind stereospecifically to a target protein, hereby interrupting specific intracellular metabolic processes that ultimately lead to cell death. Moreover, in sharp contrast to AMPs with defined secondary structure like mellitin or gramidicin S, the proline-rich peptides appear to be non-toxic to eukaryotic cells and are not haemolytic.

Drosocin is in nature glycosylated by a Gal-GalNAc disaccharide at the threonine position. In its glycosylated form it is moderately active against Gram-negative bacteria, needing 24 hours to kill bacteria *in vivo.* Unglycosylated, the action is approximately ten times less. However, when the native, glycosylated peptide is injected into mice, the glycopeptide shows no antibacterial activity, which is probably due to the rapid decomposition of the peptide in mammalian serum (Hoffmann et al., 1999, Biochem. Biophys. Acta 1426: 459-467). In mammalian serum the peptide is completely degraded within a four hour period due to both aminopeptidase and carboxypeptidase cleavage. Metabolites from drosocin which lack as few as five amino terminal or two carboxy terminal residues were inactive (Bulet et al., 1993, J. Biol. Chem. 300: 567-575; Hoffmann *et al., supra*). This observation was further supported by a model of the bioactive secondary structure of drosocin, which identifies two reverse turns, one at each terminal region, as binding sites to the target molecule (A.M. McManus et al., 1999, Biochem. 38(2): 705-714).

Interestingly, the activity spectrum of drosocin is not limited to Gram-negative species only. For instance, it has been reported that the peptide is active against the Gram-positive strain *M. luteus* in the low micromolar range. The broad spectrum of activity of drosocin forms an ideal starting point in the development of novel antimicrobial peptides. It is the goal of the present invention to develop (unglycosylated) drosocin derivatives which are more stable than the wild type drosocin in the serum of mammalians compared to the wild-type drosocin, while maintaining or increasing the antimicrobial activity as compared to native, unglycosylated drosocin.

### SUMMARY OF THE INVENTION

Surprisingly it has now been found that mutations of the N-terminal or C-terminal positions or positions 6 or 7 of drosocin yield antimicrobial peptides which have a serum half-life that is longer than that of wild-type drosocin with an identical or increased antimicrobial activity than that of wild-type drosocin.

Novel drosocin derivatives having the general formula:

R₁-Z₁-KPRP-X₁-X₂-PRPTSHPRPIR-Z₂-R₂

wherein K, P, R, T, S, H, and I are the common denominations used for lysine, proline, arginine, threonine, serine, histidine and isoleucine, respectively,
Z₁ is either a glycine residue, chosen from the group consisting of L-lysine, D-lysine, aminovaleric acid, aminobutyric acid, β-alanine and sarcosine, or absent;
Z₂ is either absent or a valine residue;
R₁ is a non-peptide spacer of 5-20 atoms length to which an antibody, peptide or fluorescent label is attached, or absent;
R₂ is a free hydroxyl, an amide or an imide moiety, a non-peptide spacer of 5-20 atoms length to which an antibody, peptide or fluorescent label is attached;
X₁ is a N-methylphenylalanine, L-tyrosine, D-tyrosine, L-phenylalanine, D-phenylalanine, N-methyl-tyrosine, or L-β3-tyrosine
X₂ is a L-serine, D-serine, N-methylserine, L-threonine, D-threonine, N-methylthreonine, or L-β3-serine
with the proviso that, when Z₁ is glycine, Z₂ is valine, R₁ is absent, R₂ is hydroxyl or amide, and X₁ is L-tyrosine, X₂ can not be L-serine.

Specifically the compound is one of the compounds 2-15 listed in Table 1.

Also part of the invention are methods to produce the abovementioned antibiotic compounds.

Further part of the invention are pharmaceutical compositions comprising one or more of the peptides of the invention, whether or not in the presence of other pharmaceutically active compounds.

Also part of the invention is the peptide according to the invention for use as a pharmaceutical and/or for the preparation of a medicament which can be used as an antibiotic.

### DETAILED DESCRIPTION OF THE INVENTION

Several peptides and peptidomimetics (also called drosocin-derivatives) have been synthesized in which one or more of the original amino acids of wild-type drosocin have been substituted by another amino acid, which can be both in the L- or the D-configuration, or even the D-isomer of the wild-type amino acid residue, or by moieties that can link to amino acids to form a chain.

Surprisingly, it now has been found that not only do these changes increase the half-life of the compounds in mammalian serum (for which they were originally designed), but also that these changes do not decrease, but sometimes even increase the intrinsic antibiotic activity when compared to the wild-type peptide.

The changes are preferably introduced at the termini of the amino acid sequence. These termini are prone to protease degradation in the serum, and appear not to be very critical for the antibiotic activity of the peptide. Increasing the 'resistance' to protease degradation increases the half-life of the peptide in the serum. Additionally, modification of the termini also allows for coupling of the peptide to other moieties, such as other amino acid sequences (thereby possibly creating multimeric proteins), or other biomolecules which can function as carrier or label. In a specific embodiment the carrier molecule also functions as a targeting molecule, which is able to localise the bacterial infection and can bind to the bacterium, in order to bring the antibiotic compound in the vicinity of the (bacterial) cell to attack. Such targeting moieties can be molecules which are known to bind to the lipopolysaccharide (LPS) molecules, which form the outside of Gram-negative bacteria. Known compounds for this use are, for instance, anchor peptides, such as the AcmA motif of Lactobacillus, or antibodies directed to lipospolysaccharide. The latter are preferred since they also have an intrinsic antibiotic effect and thus could be used for enhancing the action of the peptide of the invention.

In modifying the N-terminal amino acid of the (wild-type) peptide, it is very advantageous to insert or retain a moiety which has a positive charge under physiological circumstances, i.e. in the (human) body. Physiological circumstances thus mean a pH of about 6-8 and a temperature of about 30-40 °C. This positive charge is thought to be necessary for the antibacterial function of the wild type drosocin.

Also preferred are modifications at position 6 or 7 of the wild-type drosocin amino acid sequence. It has appeared from studies on the degradation of drosocin in mammalian sera, that the most abundant early degradation product of non-glycosylated drosocin is lacking six N-terminal residues (i.e. the proteolytic cleavage has taken place between Tyr6 and Ser7). Prevention of this endopeptidasic cleavage by changing the target residues 6 and 7 in general yields a more stable peptide (although it has been observed that sometimes increased lysis at the residues nearer to the N-terminus occurs. From the experimental results it can be gleaned that apparently the amino acids at positions 6 and 7 are also not very critical for antibiotic action, since single amino acid replacement at these positions does not greatly affect efficacy. On the contrary, sometimes the antibiotic activity is increased with respect to wild-type drosocin antibiotic activity.

The term "peptide" as used herein means a sequence of amino acids coupled by a peptide bond, wherein the amino acids are one of the twenty naturally peptide-building amino acids and wherein the amino acids can be in the L-configuration or in the D-configuration, or, for isoleucine and threonine in the D-allo configuration (only inversion at one of the chiral centers). A peptide according to the invention can be linear, i.e. wherein the first and last amino acid of the sequence have a free NH₂- or COOH-group respectively.

The term "peptide derivatives" as used herein (also called "peptidomimetics") means compounds derived from the wild-type drosocin amino acid sequence by substitution and/or modification of one or more of the amino acid residues by chemical moieties other than naturally peptide-building amino acid residues. Such chemical moieties can be non-naturally-peptide building amino acids, such as ornithine, norleucine, norvaline, statine, N-methylvaline, 6-N-methyllysine, N-methylisoleucine, N-methylserine, sarcosine, GABA, dihydroxyphenylalanine, isodesmosine, 4-hydroxyproline, 3-hydroxyproline, allo-hydroxylysine, hydroxylysine, N-ethylasparagine, N-ethylglycine, 2,3-diaminipropionic acid, 2,2'-diaminopimelic acid, desmosine, 2,4-diaminobutyric acid, 2-aminoadipic acid, 3-aminoadipic acid, β-alanine, 2-aminobutyric acid, piperidinic acid, 6-aminocaproic acid, 2-aminoheptanoic acid, 2-aminoisobutyric acid, 3-aminoisobutyric acid, aminovaleric acid, 2-aminopimelic acid and all (further) modifications to side chains of natural occurring amino acids (see for further examples e.g. Hunt, S., The non-protein amino acids. In: Chemistry and Biochemistry of the Amino Acids. Barrett, G.C. (ed.), Chapman and Hall, London, 1985). Also applicable are moieties that can form a covalent bond with both the COOH group of the preceding amino acid residue and the NH₂-group of the following amino acid residue, and which thus not necessarily need to maintain the peptide backbone structure, such as sugar amino acid dipeptide isosters, azapeptides, β-homopolymers, γ-peptides, γ-lactam analogues, oligo(phenylene ethylene)s, vinylogous sulfonopeptides, poly-N-substituted glycines, oligocarbamates and oligoureas. Further peptide derivatives can be formed by modifications at the N-terminal or C-terminal ends of the peptide. These changes can, for instance, be addition of an alkyl or alkanoyl group (either having a straight chain or being branched or cyclic or heterocyclic) or addition of a macromolecule or a reporter moiety, either via a permanent linkage or a connection that can be cleaved under certain conditions (such as disulfide bridges).

The peptides or peptide derivatives of the invention can be produced synthetically or, where applicable, recombinantly by conventional methods. Specific embodiments of drosocin-derived antibiotic peptides or peptide derivatives are disclosed in detail in the experimental part below. Preferably, the peptides or peptide derivatives of the invention are prepared conventionally by known chemical synthesis techniques, such as, for instance, are disclosed by Merrifield (J. Am. Chem. Soc. (1963) 85:2149-2154).

Alternatively, the peptides of the invention may be produced by recombinant DNA techniques by cloning and expressing within a host microorganism or cell a DNA fragment carrying a nucleic acid sequence encoding one of the above-described peptides. Nucleic acid coding sequences can be prepared synthetically, or may be derived from existing nucleic acid sequences (e.g. the sequence coding for wild-type drosocin) by site-directed mutagenesis. These nucleic acid sequences may then be cloned in a suitable expression vector and transformed or transfected into a suitable host cell, such as *E. coli, Bacillus, Lactobacillus, Streptomyces,* mammalian cells (such as CHO , HEK or COS-1 cells), yeasts (e.g. *Saccharomyces, Schizophyllum*), insect cells or viral expression systems, such as baculovirus systems. A person skilled in the art will have knowledge of the techniques of constructing the nucleic acid sequences and providing means to enable their expression.

It is also possible to include non naturally occurring amino acids in peptides through genetic engineering techniques. This has been extensively described in Noren et al., Science 244:182 (1989) and Ellman et al. Meth. Enzymol. 202:301 (1991).

Subsequently, the peptide can be isolated from the culture of the host cells. This can be achieved by common protein purification and isolation techniques which are available in the art. Such techniques may e.g. involve immunoadsorption or chromatography. It is also possible to provide the peptides with a tag (such as a histidine tag) during synthesis, which allows for a rapid binding and purification, after which the tag is enzymatically removed to obtain the active peptide.

If the peptide itself cannot be encoded or expressed but is very similar to a peptide that can be encoded or expressed, the method can be applied to prepare the peptide to which the peptide is similar, followed by one or more steps in which said peptide is modified by chemical or enzymatic techniques to prepare the final peptide or peptidomimetic.

Some more comprehensive summaries of methods which can be applied in the preparation of the peptides are described in: W. F. Anderson, Nature 392 Supp., 30 April 1998, p. 25-30; Pharmaceutical Biotechnology, Ed. D. J. A. Crommelin and R. D. Sindelar, Harwood Academic Publishers, 1997, p. 53-70, 167-180, 123-152, 8-20; Protein Synthesis: Methods and Protocols, Ed. R. Martin, Humana Press, 1998, p. 1-442; Solid-Phase Peptide Synthesis, Ed. G. B. Fields, Academic Press, 1997, p. 1-780; Amino Acid and Peptide Synthesis, Oxford University Press, 1997, p. 1-89.

The peptides according to the formula of claim 1 can be readily meade by a person skilled in the art.

The drosocin-derivatives of the invention may be used alone, or in combination in the form of multimers. Suitable combinations of peptides of the invention comprise concatemers of peptides of the invention serially coupled to each other via spacers, for instance in the form of a peptide dimer, a peptide trimer, etc., wherein the individual peptides are subsequently aligned. Single peptide or peptidomimetic chains may be coupled to a biocompatible protein, such as human serum albumin, humanized antibody, liposome, micelle, synthetic polymer, nanoparticle, and phage. Alternatively, multimers of individually combined peptides of the invention may be prepared in the form of dendrimers, or clusters, wherein three or more peptides are linked to one common centre.

Yet other combinations in the form of multimers may be formed by beads on the surface of which the peptides of the invention are exposed. The bead may then function as a carrier for the peptide, and may similarly function as a detectable label. Multimers can, for example, be prepared by biotinylating the N-terminus of peptide or peptidomimetic chains and subsequent complexation with streptavidin. As streptavidin is able to bind 4 biotin molecules or conjugates with high affinity, very stable tetrameric peptide complexes can be formed by this method. Multimers may be composed of identical or different peptides or peptidomimetics according to the invention. Preferably, however, the multimers of the invention are composed of two or more peptides or peptidomimetics, in which each component constitutes to one asset of the total biocidal activity (targeting, antimicrobial activity, scavenging).

A pharmaceutical composition of the invention comprises a therapeutically effective amount of one or more drosocin derivatives of the present invention. Once formulated, the pharmaceutical compositions of the invention can be administered directly to the subject in a method of treating bacterial infection comprising administering to a subject in need thereof a therapeutically effective amount of the composition of the invention.

Direct delivery of the compositions will generally be accomplished by topical application or other forms of administration, either orally, parenterally, subcutaneously, sublingually, intralesionally, intraperitoneally, intravenously or intramuscularly, pulmonarily, or delivered to the interstitial space of a tissue.

The pharmaceutical composition may also comprise a suitable pharmaceutically acceptable carrier or diluent and may be in the form of a capsule, tablet, lozenge, dragee, pill, droplet, suppository, powder, spray, vaccine, ointment, paste, cream, inhalant, patch, aerosol, and the like. As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the peptide or peptide conjugate.

A pharmaceutical composition may thus contain a pharmaceutically acceptable carrier. The term "pharmaceutically acceptable carrier" also includes a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Salts of peptides or functional equivalents are prepared by known methods, which typically involve the mixing of the peptide or peptidomimetic with either a pharmaceutically acceptable acid to form an acid addition salt, or with a pharmaceutically acceptable base to form a base addition salt. Whether an acid or a base is pharmaceutically acceptable can be easily decided by a person skilled in the art after taking the specific intended use of the compound into consideration. For instance, not all acids and bases that are acceptable for *ex vivo* applications can be used for therapeutic compositions. Depending on the intended use, pharmaceutically acceptable acids include organic and inorganic acids such as formic acid, acetic acid, propionic acid, lactic acid, glycolic acid, oxalic acid, pyruvic acid, succinic acid, maleic acid, malonic acid, cinnamic acid, sulfuric acid, hydrochloric acid, hydrobromic acid, nitric acid, perchloric acid, phosphoric acid, and thiocyanic acid, which form ammonium salts with free amino groups of peptides and functional equivalents. Pharmaceutically acceptable bases, which form carboxylate salts with free carboxylic groups of peptides and functional equivalents, include ethylamine, methylamine, dimethylamine, triethylamine, isopropylamine, diisopropylamine, and other mono-, di- and trialkylamines, as well as arylamines. Moreover, also pharmaceutically acceptable solvates are encompassed.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

For therapeutic treatment, peptide or peptide-conjugate may be produced as described above and applied to the subject in need thereof. The peptide or peptide-conjugate may be administered to a subject by any suitable route, preferably in the form of a pharmaceutical composition adapted to such a route and in a dosage which is effective for the intended treatment.

Pharmaceutical compositions of this invention may contain other active agents, such as conventional antibiotics (like e.g. vancomycin, streptomycin, tetracyclin, penicillin) or other antimicrobial compounds, such as anti-fungals, e.g. itraconazole or myconazole. Also compounds which alleviate other infection symptoms, such as fever (e.g. salicylic acid) or skin rash may be added.

Therapeutically effective dosages of the peptide, peptidomimetic or peptide-conjugate required for treating a bacterial infection in the body of a human or animal subject, can easily be determined by the skilled person, for instance by using animal models.

The term "therapeutically effective amount" as used herein refers to an amount of a therapeutic, *viz.* a peptide, peptidomimetic or peptide-conjugate according to the present invention, to reduce or prevent growth and colonization of bacteria, or to exhibit a detectable therapeutic or prophylactic effect. The effect can be detected by, for example, culturing biopsies and assaying for bacterial activity or by any other suitable method of assessing the progress or severity of bacterial infection. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Specifically, the compositions of the present invention can be used to reduce or prevent bacterial infection and/or accompanying biological or physical manifestations, such as reduction of fever. Methods that permit the clinician to establish initial dosages are known in the art. The dosages determined to be administered must be safe and efficacious.

For purposes of the present invention, an effective dose will be from about 0.01 µg/kg to 50 mg/kg, preferably 0.5 µg/kg to about 10 mg/kg of the peptide, peptidomimetic or peptide-conjugate in the individual to which it is administered. Dosages for achieving the therapeutic effects of the pharmaceutical composition described herein may easily be determined by the skilled person.

Yet in another alternative embodiment, the peptide, peptidomimetic or peptide-conjugate or compositions of the invention may be administered from a controlled or sustained release matrix inserted in the body of the subject.

It may also be advantageous to administer a compound of the invention in a transmucosal dosage form. This route of administration is non-invasive and patient-friendly; at the same time it may lead to an improved bioavailability of the compound compared to oral administration, especially if the compound is not stable in the fluids of the digestive system, or if it is too large to be absorbed from the gut effectively. Transmucosal administration is possible, for instance, via nasal, buccal, sublingual, gingival, or vaginal dosage forms. These dosage forms can be prepared by known techniques; they can be formulated to represent nasal drops or sprays, inserts, films, patches, gels, ointments, or tablets. Preferably, the excipients used for a transmucosal dosage form include one or more substances providing for mucoadhesion, thus prolonging the contact time of the dosage form with the site of absorption and thereby potentially increasing the extent of absorption.

In a further embodiment, the compounds are administered via the pulmonary route, using a metered dose inhaler, a nebulizer, an aerosol spray, or a dry powder inhaler. Appropriate formulations can be prepared by known methods and techniques. Transdermal, rectal, or ocular administration may also be feasible in some cases.

It can be advantageous to use advanced drug delivery or targeting methods to deliver a compound of the invention more effectively. For instance, if a non-parenteral route of administration is chosen, an appropriate dosage form may contain a bioavailability enhancing agent, which may be any substance or mixture of substances which increases the availability of the compound. This may be achieved, for instance, by the protection of the compound from degradation, such as by an enzyme inhibitor or an antioxidant. More preferably, the enhancing agent increases the bioavailability of the compound by increasing the permeability of the absorption barrier, which is typically a mucosa. Permeation enhancers can act via various mechanisms; some increase the fluidity of mucosal membranes, while others open or widen the gap junctions between mucosal cells. Still others reduce the viscosity of the mucus covering the mucosal cell layer. Among the preferred bioavailability enhancers are amphiphilic substances such as cholic acid derivatives, phospholipids, ethanol, fatty acids, oleic acid, fatty acid derivatives, EDTA, carbomers, polycarbophil, and chitosan.

Indications for which the drosocin derivatives of the invention can be used are bacterial infections by both Gram-positive and Gram-negative bacteria, such as *E. coli, Agrobacterium tumefaciens, Salmonella typhimurum, Erwinia carotovora, E. herbicola, E. chrysanthemi, Klebsiella pneumoniae, Haemophilus influenzae, Francisella tularensis, Bacillus anthracis, Bacillus megaterium, Clostridium botulinum, Brucella* spp., *Coxiella burnetii, Yersinia pestis, Listeria monocytogenes, Mycobacterium tuberculosis, Pasteurella aeruginosa, Pneumococcus spp., Salmonella* spp., *Streptococcus* spp., *Staphylococcus aureus, Staphylococcus pyrogenes, Micrococcus luteus, Moraxella, Neisseria ghonnorhoea, Aerobacter, Borellia.*

Next to therapeutic use for treatment of infections, also in biological warfare, it is also possible to use the antiobiotic peptides of the invention in a bactericidal composition which can be used to clean surfaces and/or equipment. Another field of application is in packaging, where peptides can be linked to or embedded in packaging material for packaging of food or other material which is easily degradable by micro-organisms. The drosocin derivatives of the invention are specifically usable for packaging, since they are not toxic upon contact or ingestion.

### EXAMPLES

### Example 1

**Solid Phase Peptide Synthesis.** Peptides were synthesized on 10 µmol scale. The first amino acid, Fmoc-Val-OH (10eq.), was manually coupled to Wang resin (0.86 mmol/g) with DIC (10 eq.) and DMAP (0.1 eq.) in DMF for 2 h. Loading was determined by UV-determination of the amount of Fmoc-chromophore released after treatment with 20 % piperidine/DMF for 10 min (0.51 mmol/g). Automated peptide synthesis was continued with 5 eq. amino acid, using customized programs, comprising single couplings, deprotection of the Fmoc group with 20 % piperidine in NMP, and capping of unreacted sequences by acetylation (Ac₂O, DIEA, DMF). N-Methyl amino acids (4 eq.) were coupled manually with BOP/HOBt/DIEA/DMF. Amino acids to be coupled onto N-methyl amino acids were also coupled manually, using the amino acid (5 eq.) and preactivation with HATU or PyBroP (5 eq.) and DIEA (10eq.) in DMF. If chloranil test was positive, coupling was repeated. Fmoc-SAA-OH **13** was double coupled with 7.5 eq. as well as the following amino acid (Pro5 at 5 eq). The sequences were finished through automated synthesis. After removal of the last Fmoc-group, crude peptides were treated with TFA/TIS/H₂O (95/2.5/2.5, v/v/v) for 1.5 h to remove all protecting groups and simultaneously cleave the peptides from the resin. Crude peptides were precipitated in Et₂O and centrifuged. After decantation of the solvents, materials were lyophilized, analyzed with LC-MS, and purified on a HPLC system using gradients of MeCN/H₂O + 0.1% TFA. Yields typically varied between 5-13 % and the compounds were characterized as shown in Table 2.

### Example 2

The serum stability studies were carried out in triplicate (results in Table 2). Briefly, 10 µL of an aqueous peptide solution (0.8 mg/mL) was added to 1 mL freshly pooled 25% human serum (Sigma, St. Louis, MO, USA) in PBS. The mixtures were thermostated at 37 °C under gentle stirring. At t= 0, 1, 2, 4, 6 and 8 h, 100 µL of each mixture was taken out and precipitated in 230 µL 15% aqueous TCA. Samples were stored at 0 °C for 20 min and centrifuged (4000 rpm) for 5min at 0 °C. The supernatants (250 µL of each) were immediately stored at -80°C until analyzed by MALDI-TOF mass spectrometry. The spectra were recorded in the linear mode, using α-cyanohydroxycinnamic acid in 50% CH₃CN in 0.1% aqueous TFA. Serum control samples consisted of 100 µL of pooled 25% human serum solution in PBS precipitated in 230 µL 15% aqueous TCA. Peptide reference samples consisted of 10 µL of the peptide stock solution diluted in 1 mL H₂O of which 100 µL was added to 230 µL 15% aqueous TCA.

### Example 3

Antibacterial assays were performed in sterilized round-bottom 96-well plates (polystyrene, U-bottom, Costar) with a final volume of 110 µL as follows (results in Table 1). The bacteria (*E. coli* ATCC 11775) were grown in nutrient broth E (NB, Oxoid, UK, beef extract 1 g/L, yeast extract 2 g/L, peptone 5 g/L and NaCl 5 g/L) and kept at 4 °C. Lyophilized peptides were dissolved in NB to give a concentration of 400 µM and filtered using 0.22 µm filter discs (Millex GV, Durapore). An overnight culture in NB was adjusted to 5 × 10⁶ CFU/mL and inoculated into the micro titer plate wells(10 µL) containing each 100 µL of a serial 2-fold dilution (200-0.4 µM) of the tested peptide in NB. Plates were incubated while gently shaking at 37 °C for 24 h. Next, 80 µL suspension of each well was transported into a flat-bottom 96-well plate (Costar). The absorbance was measured at 600 nm using a µQuant micro plate spectrophotometer (Bio-Tek Instruments). All peptides were measured in quadruplo. The peptide with sequence NTDGSTDYGILQINSR (see Otvos Jr, L.; Bokonyi, K.; Varga, I; Otvos, B.I.; Hoffmann, R.; Ertl, H.C.J.; Wade, J.D.; McManus, A.M.; Craik, D.J.; Bulet, P. Protein Sci. 2000, 9, 742) was used as negative control.

### Example 4

Additional antimicrobial tests have been carried out for a number of drosocin derivatives; for results see Table 3. Yersinia pestis KIM5 (BM351), Y. pseudotuberculosis (BM115), Vibrio cholerae (BM301), Bacillus anthracis (BM233),Bacillus anthracis (BM013), Escherichia coli O157 H7 (BM175), Methicillin Resistant Staphylococcus aureus (BM253), Shigella sonnei (ATCC 11060/ BM345), Pseudomonas auruginosa (BM179), Serratia marcescens (BM107), Enterobacter aerogenes (BM177), Klebsiella pneumoniae (BM178), Erwinia herbicola (BM089), E. chrysanthemi (Ech502), E. carotovora atroseptica (Eca161), Salmonella montevideo (BM180), S. give (BM241), S. enteritidis (BM256), S. infantis (BM242), S. panama (BM243), S. typhimurium (BM255), S. typhimurium (BM352), S. typhimurium Rb chemotype (BM353), S. typhimurium Rc chemotype (BM354), S. typhimurium Rd1 chemotype (BM355), S. typhimurium Re chemotype (BM356), Moraxella catarrhalis (BM267), Clostridium botulium type A (BM272), Neisseria meningitidisi (BM268), Streptococcus feacalis (BM189), Streptococcus pneumoniae (BM263), were grown in nutrient broth (NB, Oxoid, UK, beef extract 1 g/L, yeast extract 2 g/L, peptone 5 g/L and NaCl 5 g/L) or Brain heart infusion (BHI 37 g/L (LAB M, Bury, UK). MIC values were determined as described above (Example 3).

**Table 1. Selected analytical data and antibacterial activities of peptides 1-15**

| Peptide | Sequence^{†} | MIC^{§} (µM) |
|---|---|---|
| | | |
| 1 | GKPRP YSPRP TSHPR PIRV | 6.3 |
| 2 | GKPRP Y^{Me}SPRP TSHPR PIRV | 25 |
| 3 | GKPRP FSPRP TSHPR PIRV | 3.1 |
| 4 | GKPRP YTPRP TSHPR PIRV | 1.6 |
| 5 | GKPRP FTPRP TSHPR PIRV | 3.1 |
| 6 | GKPRP DYSPRP TSHPR PIRV | 6.3 |
| 7 | GKPRP YDSPRP TSHPR PIRV | 3.1 |
| 8 | GKPRP DYDSPRP TSHPR PIRV | 25 |
| 9 | GKPRP βYSPRP TSHPR PIRV | 3.1 |
| 10 | GKPRP YβSPRP TSHPR PIRV | 3.1 |
| 11 | GKPRP βYβSPRP TSHPR PIRV | 12.5 |
| 12 | AvaKPRP YTPRP TSHPR PIRV | 6.3 |
| 13 | AbuKPRP YTPRP TSHPR PIRV | 6.3 |
| 14 | βAlaKPRP YTPRP TSHPR PIRV | 3.1 |
| 15 | SarKPRP YTPRP TSHPR PIRV | 6.3 |

| | | |
|---|---|---|
| ^{†} modifications are highlighted in boldface, wherein ^{Me}S denotes *N*-methyl serine, D indicates the D-configuration of the respective amino acid, β indicates the L-β³-homocounterpart of the respective amino acid, Ava= aminovaleric acid, Abu= amino butyric acid, βAla= 6-alanine and Sar= sarcosine (*N*-methylglycine); ^{§} MIC was defined as the lowest peptide concentration that prevented bacterial growth of *E*. *coli* ATCC 11775 after 24 h of incubation at 37 °C in nutrient broth.E, with 5 × 10⁶ CFU/mL; | | |

**Table 2**

| Peptide¹ | Intact after 8 hr (%) | index² |
|---|---|---|
| **1** | 3 | 1.0 |
| **2** | 11 | 0.9 |
| **3** | 19 | 12 |
| **4** | 15 | 19 |
| **5** | 26 | 17 |
| **6** | 16 | 5 |
| **7** | 1 | 0.6 |
| **8** | 17 | 1 |
| **9** | 2 | 1 |
| **10** | 31 | 20 |
| **11** | <1 | 0.001 |

| | | |
|---|---|---|
| ¹Peptide indicated by the same number as in Table 1. ²Index for peptide X is calculated as follows: [MIC(1)/MIC(**X**)] x [%intact(**X**)/% intact(**1**)] | | |

**Table 3.**

| Strain | Gram | Growth medium | Growth Temp. (°C) | MIC (µM) Drosocin^{a} | MIC (µM) drosocin S7T^{b} | MIC (µM) drosocin B-Ala + S7T^{c} |
|---|---|---|---|---|---|---|
| *Yersinia pestis* | - | BHI | 22 | NI at ≤ 100 | NI at ≤ 100 | NI at ≤ 100 |
| KIM5 | | | | | | |
| *Yersinia* | - | BHI | 35 | NI at ≤ 100 | NI at ≤ 100 | NI at ≤ 100 |
| *pseudotuberculosis* | | | | | | |
| *Vibrio cholera* | - | BHI | 37 | NI at ≤ 100 | NI at ≤ 100 | NI at ≤ 100 |
| *Bacillus anthracis* | + | BHI | 37 | NI at ≤ 100 | NI at ≤ 100 | NI at ≤ 100 |
| *Bacillus globigii* | + | | 37 | NI at ≤ 100 | NI at ≤ 100 | NI at ≤ 100 |
| *Bacillus* | + | BHI | 37 | NI at ≤ 100 | NI at ≤ 100 | NI at ≤ 100 |
| *thuringiensis* | | | | | | |
| *Pseudomonas* | - | BHI | 35 | NI at ≤ 100 | NI at ≤ 100 | NI at ≤ 100 |
| *auruginosa* | | | | | | |
| *Methicillinresistant* | + | NB | 37 | NI at ≤ 100 | NI at ≤ 100 | NI at ≤ 100 |
| *Staphylococcus* | | | | | | |
| *aureus (MRSA)* | | | | | | |
| *Serratia marcescens* | - | NB | 22 | NI at ≤ 100 | NI at ≤ 100 | NI at ≤ 100 |
| *Erwinia carotovora* | - | NB/BHI | 28/37 | | | |
| *atroseptica* | | | | NI at ≤ 100 | NI at ≤ 100 | NI at ≤ 100 |
| *Enterobacter* | - | NB | 37 | 100 | 100 | 100 |
| *aerogenes* | | | | | | |
| *Klebsiella* | - | NB | 37 | 100 | 100 | 100 |
| *pneumoniae* | | | | | | |
| *Salmonella* | - | NB | 37 | | | |
| *montevideo* | | | | 50 | 12.5 | 12.5 |
| *Salmonella give* | - | NB | 37 | 25 | 25 | 100 |
| *Salmonella infantis* | - | NB | 37 | 50 | 25 | 25 |
| *Salmonella panama -* | | NB | 37 | 25 | 12.5 | 25 |
| *Salmonella* | - | NB | 37 | | | |
| *typhimurium* | | | | 25 | 12.5 | 50 |
| *Salmonella* | - | NB | 37 | | | |
| *enteritidis* | | | | 50 | 12.5 | 12.5 |
| *Erwinia herbicola* | - | NB | 37 | 12.5 | 6.75 | 6.75 |
| *Erwinia* | - | NB | 28 | | | |
| *chrysanthemi* | | | | 100 | 50 | 100 |
| *E. coli* O157 :H7 | - | NB | 37 | 25 | 12.5 | 25 |
| *Shigella* sonnei | - | BHI | 37 | 100 | 100 | 100 |
| *Salmonella* | - | NB | | | | |
| *typhimurium* Rb | | | 37 | 50 | 12.5 | 12.5 |
| *Salmonella* | - | NB | | | | |
| *typhimurium* Rc | | | 37 | 50 | 100 | 100 |
| *Salmonella* | - | NB | | | | |
| *typhimurium* Rd1 | | | 37 | 25 | 12.5 | 12.5 |
| *Salmonella* | - | NB | | | | |
| *typhimurium* Re | | | 37 | 6.75 | 3.38 | 3.38 |

| | | | | | | |
|---|---|---|---|---|---|---|
| NI at ≤ 100: Not inhibited at peptide concentrations ≤ 100µM ^{a}GKRPRP YSPRP TSHPR PIRV ^{b}GKRPRP YTPRP TSHPR PIRV ^{c}β-Ala-GKRPRP YTPRP TSHPR PIRV | | | | | | |

## Claims

1. Drosocin derivative having the general formula:
R₁-Z₂-KPRP-X₁-X₂-PRPTSHPRPIR-Z₂-R₂
wherein K, P, R, T, S, H, and I are the common denominations used for lysine, proline, arginine, threonine, serine, histidine and isoleucine, respectively,
Z₁ is either a glycine residue, an amino acid chosen from the group consisting of L-lysine, D-lysine, aminovaleric acid, aminobutyric acid, β-alanine and sarcosine, or absent;
Z₂ is either absent or a valine residue;
R₁ is a non-peptide spacer of 5-20 atoms length to which an antibody, peptide or fluorescent label is attached, or absent;
R₂ is a free hydroxyl, an amide or an imide moiety, a non-peptide spacer of 5-20 atoms length to which an antibody, peptide or fluorescent label is attached;
X₁ is a N-methylphenylalanine, L-tyrosine, D-tyrosine, L-phenylalanine, D-phenylalanine, N-methyl-tyrosine, or L-β3-tyrosine
X₂ is a L-serine, D-serine, N-methylserine, L-threonine, D-threonine, N-methylthreonine, or L-β3-serine
with the proviso that, when Z₁ is glycine, Z₂ is valine, R₁ is absent, R₂ is hydroxyl or amide, and X₁ is L-tyrosine, X₂ can not be L-serine .

2. Drosocin derivative according to claim 1, wherein the compound is selected from the group of compounds consisting of nrs. 2-15 of Table 1.

3. Pharmaceutical composition comprising at least one drosocin derivative according to claim 1 or 2.

4. Drosocin derivative according to claim 1 or 2 for use in the treatment of bacterial infections.

## Patentansprüche

1. Drosocinderivat mit der allgemeinen Formel
R₁-Z₁-KPRP-X₁-X₂-PRPTSHPRPIR-Z₂-R₂
in der K, P, R, T, S, H und I die üblicherweise für Lysin, Prolin, Arginin, Threonin, Serin, Histidin und isoleucin verwendeten Bezeichnungen sind,
Z₁ entweder ein Glycinrest, eine Aminosäure ausgewählt aus der Gruppe bestehend aus L-Lysin, D-Lysin, Aminovaleriansäure, Aminobuttersäure, β-Alanin und Sarcosin ist, oder nicht vorhanden ist,
Z₂ entweder nicht vorhanden ist oder ein Valinrest ist,
R₁ eine Abstandsgruppe mit 5 bis 20 Atomen Länge ist, die kein Peptid ist und an die ein Antikörper, Peptid oder Fluoreszenzmarker gebunden ist, oder nicht vorhanden ist, R₂ eine freie Hydroxylgruppe, ein Amid- oder ein Imidanteil, oder eine Abstandsgruppe mit 5 bis 20 Atomen Länge ist, die kein Peptid ist und an die ein Antikörper, Peptid oder Fluoreszenzmarker gebunden ist,
X₁ ein N-Methylphenylalanin, L-Tyrosin, D-Tyrosin, L-Phenylalanin, D-Phenylalanin, N-Methyltyrosin oder L-β3-Tyrosin ist,
X₂ ein L-Serin, D-Serin, N-Methylserin, L-Threonin, D-Threonin, N-Methylthreonin oder L-β3-Serin ist,
mit der Maßgabe, dass X₂ nicht L-Serin sein kann, wenn Z₁ Glycin ist, Z₂ Valin ist, R₁ nicht vorhanden ist, R₂ Hydroxyl oder Amid ist und X₁ L-Tyrosin ist.

2. Drosocinderivat nach Anspruch 1, bei dem die Verbindung ausgewählt ist aus der Gruppe bestehend aus den Nummern 2 bis 15 von Tabelle 1.

3. Pharmazeutische Zusammensetzung, die mindestens ein Drosocinderivat gemäß Anspruch 1 oder 2 enthält.

4. Drosocinderivat nach Anspruch 1 oder 2 zur Verwendung bei der Behandlung von bakteriellen Infektionen.

## Revendications

1. Dérivé de drosocine de formule générale:
R₁-Z₁-KPRP-X₁-X₂-PRPTSHPRPIR-Z₂-R₂
où K, P, R, T, S, H et I sont les dénominations communément utilisées pour désigner respectivement la lysine, la proline, l' arginine, la thréonine, la sérine, l'histidine et l'isoleucine,
Z₁ est soit un résidu de glycine, un acide aminé choisi dans le groupe formé par la L-lysine, la D-lysine, l'acide aminovalérique, l'acide aminobutyrique, la β-alanine et la sarcosine, soit absent;
Z₂ est soit absent, soit un résidu de valine ;
R₁ est un espaceur non peptidique de 5 à 20 atomes de longueur auquel est fixé un anticorps, un peptide ou un traceur fluorescent, ou est absent ;
R₂ est un hydroxyle libre, un amide ou une fonction imide, un espaceur non peptidique de 5 à 20 atomes de longueur auquel est fixé un anticorps, un peptide ou un traceur fluorescent ;
X₁ est une N-méthylphénylalanine, L-tyrosine, D-tyrosine, L-phénylalanine, D-phénylalanine, N-méthyl-tyrosine ou L-β3-tyrosine;
X₂ est une L-sérine, D-sérine, N-méthylsérine, L-thréonine, D-thréonine, N-méthylthréonine ou L-β3-sérine;
à la condition que, lorsque Z₁ est la glycine, Z₂ est la valine, R₁ est absent, R₂ est un hydroxyle ou un amide et X₁ est la L-tyrosine, X₂ ne peut pas être la L-sérine.

2. Dérivé de drosocine selon la revendication 1, dans lequel le composé est choisi dans le groupe de composés formé par les numéros 2 à 15 du Tableau 1.

3. Composition pharmaceutique contenant au moins un dérivé de drosocine selon la revendication 1 ou la revendication 2.

4. Dérivé de drosocine selon la revendication 1 ou la revendication 2 destiné à être utilisé pour le traitement d'infections bactériennes.
